# EUROPEAN PATENT APPLICATION

(11) **EP 2 526 976 A2**
(43) Date of publication of application: **28.11.2012**
(21) Application number: 11175963.5
(22) Date of filing: 29.07.2011
(51) Int. Cl.: A61L 27/06, A61L 27/30, A61L 27/50

(54) **A medical implant and a method of formation of multi-phase surface layers on medical implant made from titanium and/or titanium alloys**

(30) Priority: 29.11.2010 PL 39304710
(71) Applicant: Fundacja Rozwoju Kardiochirurgii Im. Prof. Zbigniewa Religi, 41-800 Zabrze (PL)
(72) Inventor: Wierzchon, Tadeusz, 02-642 Warszawa (PL); Czarnowska, Elzbieta, 04-744 Warszawa (PL); Ossowski, Maciej, 03-284 Warszawa (PL); Gonsior, Malgorzata, 41-806 Zabrze (PL); Kustosz, Roman, 41-800 Zabrze (PL)
(74) Representative: Malcherek, Piotr

(57) **Abstract**

The invention relates to a method of formation of multi-phase surface layers on medical implants made from titanium and/or titanium alloys. First, titanium and/or titanium alloys undergo glow discharge plasma assisted nitriding within the temperature range between 580°C - 850°C until TiN + Ti₂N + αTi(N) layers are formed, then -during the same technological process - after changing the composition of the reactive atmosphere they undergo the process of glow discharge plasma assisted oxidation within the temperature range of 450°C - 700°C with air and nitride mixture where the air content is 10% volume until the TiO₂ layer is formed. The invention also relates to a medical implant containing TiO₂ layers of nano-crystalline or amorphous structure, TiN of nano-crystalline or microcrystalline structure, Ti₂N i αTi(N).

## Description

A medical implant and a method of formation of multi-phase surface layers on medical implants made from titanium and/or titanium alloys

The invention relates to a medical implant and a method of formation of multi-phase surface layers on medical implants made from titanium and/or titanium alloys, especially on medical implants designed to have contact with blood, in particular heart valve rings or elements of heart prostheses.

Titanium and its alloys are widely used in medicine, for example as a material used to manufacture components of heart prostheses or heart valve and/or its components. On the surface of such prostheses titanium oxides layers are formed spontaneously, their thickness does not exceed 6 nm; they do not prevent metallosis - penetration of titanium alloys including titanium into the surrounding tissues. Moreover, the layers do not sufficiently reduce thrombosis which is platelet deposition and platelet agglomerate creation and platelet activation on the surface of the implants even after they have been mechanically or electrochemically polished.

There are known methods of producing titanium oxide coatings or titanium nitride coatings. Among these methods is the PVD method which is a physical vapour deposition, the CVD method which is chemical vapour deposition or an electrochemical oxidation method. The coatings created as a result of using the above methods do not fully eliminate metallosis. What is more the above mentioned methods do not allow to form diffusion layers or do not ensure the formation of layers on implants with complex shapes. They are adhesive layers which means their adhesion is lower than that of diffusion layers.

There is also known a method of glow discharge plasma assisted nitriding within the temperature range of ≥ 850 °C which leads to the formation of diffusion layers of the type TiO₂+TiN+Ti₂N+αTi(N). Such temperature range of glow discharge plasma assisted nitriding process results in substantial changes to the titanium alloy microstructure especially dual-phase, in particular in grain growth, which affects the mechanical properties in a negative way, especially their fatigue strength.

The invention relates to a medical implant wholly or partially made from titanium and/or titanium alloy. The essence of the invention is that parts of the implant designed to have contact with blood are diffusion layers comprising the following layers in the following order in the direction from the outside surface:
- TiO₂ of nanocrystalline or amorphous structure ;
- TiN of nanocrystalline or micro-crystalline structure,
- Ti₂N;
- αTi(N),
where the above layers are formed in a single technological process; diffusion layers TiN + Ti₂N + αTi(N) are formed during the first stage of the process of glow discharge plasma assisted nitriding within the temperature range between 580°C - 850°C and the surface layer TiO₂ is formed during the second stage of the process of glow discharge plasma assisted oxidation within the temperature range between 450°C - 700°C with air and nitride mixture where the maximum air content is 10% volume.

In a preferred embodiment of the invention the maximum thickness of the TiO₂ layer is 400 nm whereas the diffusion layers TiN + Ti₂N + αTi(N) are formed in the process of glow discharge plasma assisted nitriding within the temperature range between 600°C - 730°C, most preferably 680°C.

Another invention relates to the method of formation of multi-phase surface layers on medical implants made from titanium and/or titanium alloys where titanium and/or titanium alloys undergoes glow discharge plasma assisted nitriding. The essence of the invention is that first titanium and/or titanium alloys undergo glow discharge plasma assisted nitriding within the range of temperature between 580°C - 850°C until TiN + Ti₂N + αTi(N) layers are formed and then after changing the reactive atmosphere they undergo the glow discharge plasma assisted oxidation process within the temperature range between 450°C - 700°C with the air and nitride mixture where the maximum air content is 10% volume until TiO₂ coating is created.

In a preferred embodiment the process of glow discharge plasma assisted nitriding is performed within the temperature range between 600°C-730°C, most preferably at 680 ° C.

It is also recommendable that during the process of glow discharge plasma assisted nitriding and/or glow discharge plasma assisted oxidation the titanium and/or titanium alloy medical implant is on the so-called plasma potential which is the low-temperature plasma formed as a result of glow discharge between the anode and the cathode.

The method according to the invention where the medical implant containing titanium and/or titanium alloys undergoes the process of glow discharge plasma assisted nitriding and then glow discharge plasma assisted oxidation in the changed composition of the reactive atmosphere during a single process allows the formation of diffusion layers on the surface of titanium and/or titanium alloys which diffusion layers have fully controlled microstructure, surface topography, have a diffusional character, homogenous thickness on parts with complex shapes, they are highly resistant to corrosion and frictional wear, they also have reduced thrombogenicity and eliminate metallosis. Medical implants produced according to the method above may be used especially in the elements of heart prostheses with longterm use. The new method allows to meet strict dimension tolerances of the machined titanium implants, the formation of nanocrystalline, relatively amorphous microstructure of titanium dioxide - TiO₂ and titanium nitride - TiN constituting the outside zones of the composite layer, the thickness of each zone of the layer and their surface topography. The temperatures used in the process permit the formation of layers of maximum 20 µm depending on the time of machining, and the temperature leads only to minor changes in the microstructure of the machined titanium alloys, therefore the advantageous mechanical properties thereof are retained with the simultaneous increase in hardness and wear resistance.

The medical implant and the method according to the invention are shown in detail in the following embodiment.

The method relates to the formation of layers of the TiO₂+TiN+Ti₂N₊αTi(N) type in the low-temperature process of glow discharge plasma assisted oxynitriding - the phase composition from the surface - on the alloy Ti6A14V used in the heart valve rings to reduce thrombosis. The heart valve rings made from Ti6A14V alloy, after polishing the surface - Rₐ= 0,19 µm, and after cleaning with ethanol underwent the process of glow discharge plasma assisted nitriding with the following parameters: temperature T = 700 °C, pressure in the working chamber - p = 2 mbar, reactive atmosphere-nitrogen - N₂ of purity level 99,99 %, time t = 4 h. The diffusion layer of the titanium nitride type - TiN+Ti₂N+αTi(N) was formed, the homogenous thickness thereof being 6 µm and the outside thickness of the titanium nitride zone - TiN app. 2,0 µm. The titanium nitride zone - TiN has nanocrystalline structure with the size of crystallites of app. 30 nm and the surface roughness of Rₐ = 0,658 nm. The formed nitrided layer has the level of hardness at 1400 HV0,05 and is highly resistant to frictional wear, substantially higher than that of titanium alloy Ti6A14V, it is highly corrosion resistant, biocompatible and eliminates metallosis which is penetration of titanium alloy components into the surrounding biological environment.

Next, after changing the composition of the reactive atmosphere, which means - by introducing app. 5% of air and decreasing the process temperature to 680 °C, during a 10-minute period in the process of glow discharge plasma assisted oxidation of titanium nitride TiN - a 200 nm thin, homogenous, nanocrystalline titanium dioxide rutile layer was formed. After the process a homogenous, diffusion composite layer of the TiO₂ type was formed, nanocrystalline, with the thickness of 200 nm + TiN, nanocrystalline, + Ti₂N + αTi(N) with the level of hardness at 1100HV0,02 and surface topography Rₐ =0,735nm, highly resistant to frictional wear and corrosion, significantly reducing thrombosis and the activation of platelets, especially after the gas sterilisation in ethylene oxide.

It is advantageous when during the process of glow discharge plasma assisted nitriding and/or glow discharge plasma assisted oxidation the titanium and/or titanium alloy medical implant is on the so-called plasma potential which is the low-temperature plasma formed as a result of glow discharge between the anode and the cathode.

## Claims

1. A medical implant wholly or partially made from titanium and/or titanium alloy, **characterised in that** parts of the surface of the implant designed to have contact with blood are diffusion layers comprising the following layers in the following order in the direction from the outside surface:
- TiO₂ of nanocrystalline or amorphous structure ;
- TiN of nanocrystalline or micro-crystalline structure,
- Ti₂N;
- αTi(N),
where the above layers are formed in a single technological process; the diffusion layers TiN + Ti₂N + αTi(N) are formed during the first stage of the process of glow discharge plasma assisted nitriding within the temperature range between 580°C - 850°C and the surface layer TiO₂ is formed during the second stage of the process of glow discharge plasma assisted oxidation within the temperature range between 450°C - 700°C with air and nitride mixture where the maximum air content is 10% volume.

2. The medical implant according to claim 1, **characterised in that** the maximum thickness of the TiO₂ layer is 400 nm.

3. A method of formation of multi-phase surface layers on medical implants made from titanium and/or titanium alloys where titanium and/or titanium alloys undergoes glow discharge plasma assisted nitriding, **characterised in that** initially titanium and/or titanium alloys undergo glow discharge plasma assisted nitriding within the range of temperature between 580°C - 850°C until TiN + Ti₂N + αTi(N) layers are formed and then after changing the reactive atmosphere they undergo the glow discharge plasma assisted oxidation process within the temperature range between 450°C - 700°C with the air and nitride mixture where the maximum air content is 10% volume until TiO₂ coating is created.

4. The method according to claim 3, **characterised in that** the process of glow discharge plasma assisted nitriding is performed within the temperature range between 600°C - 730°C, most preferably at 680 °C.

5. The method according to claim 3 or 4, **characterised in that** during the process of glow discharge plasma assisted nitriding and/or glow discharge plasma assisted oxidation the titanium and/or titanium alloy medical implant is on the so-called plasma potential which is the low-temperature plasma formed as a result of glow discharge between the anode and the cathode.
